# EUROPEAN PATENT SPECIFICATION

(11) **EP 2 340 795 B1**
(45) Date of publication and mention of the grant of the patent: **07.03.2018**
(21) Application number: 09810046.4
(22) Date of filing: 28.08.2009
(51) Int. Cl.: A61F 13/32, A61F 13/26

(54) **APPLICATOR FOR TAMPON**
TAMPON-APPLIKATOR
APPLICATEUR DE TAMPONS

(30) Priority: 29.08.2008 JP 2008222472; 29.08.2008 JP 2008222478; 29.08.2008 JP 2008222575
(43) Date of publication of application: 06.07.2011
(73) Proprietor: Unicharm Corporation, Ehime 799-0111 (JP)
(72) Inventor: WATANABE, Hitoshi, Kagawa 769-1602 (JP); KIKUCHI, Akie, Kagawa, 769-1602 (JP)
(74) Representative: Murgatroyd, Susan Elizabeth
(86) International application number: PCT/JP2009/065092
(87) International publication number: WO 2010/024398

(56) References cited:
- EP-A2- 1 832 265
- WO-A1-95/08312
- JP-B2- 3 489 833
- JP-B2- 3 998 462
- JP-T- 7 500 746
- JP-T- 2001 519 676
- JP-T- 2002 531 173
- US-B1- 6 196 988

## Description

### [Technical Field]

The present invention relates to a tampon applicator which includes an outer tube and a push-out portion, the outer tube having: a large-diameter section for storing a tampon; and a small-diameter section which is smaller in diameter than the large-diameter section that is positioned at a base portion side more than the large-diameter section, the push-out portion being adapted to press the tampon that is stored in the large-diameter section from a backward side to be able to be pushed out from an opening portion of a front end of the outer tube.

### [Background Art]

Conventionally, there is known a tampon applicator of which outer tube is covered with an absorptive sheet which is capable of absorbing and retaining menstrual blood in order to disallow a circumstance that a user's hands are contaminated by the menstrual blood adhering to a surface of such tampon applicator when the user has pulled out the tampon applicator from her vaginal cavity (refer to Patent Document 1).

### [Prior Art Document]

### [Patent Document(s)]

[Patent Document 1]
   Japanese Patent No. 3998462
[Patent Document 2]
   Japanese Patent No. 3489833

### [Summary of the Invention]

However, the abovementioned tampon applicator is constituted so that an outer tube thereof is covered with the abovementioned absorptive sheet; and therefore, manufacturing cost increases with an increased number of members, there is a danger that part of such absorptive sheet remains in virginal cavity, and there has been a problem that an outer diameter of the outer tube increases.

US 6196988 B1 and EP 1832265 A2 both disclose a tampon applicator having a barrel for storing a tampon and a plunger for pushing the tampon out of an opening provided in the insertion end of the barrel. The barrel of US 6196988 B1 has a plurality of slots to enhance the flexibility of the barrel, and the barrel of EP1832265A2 is provided with one or more embossments for improving grippability of the barrel. WO 95/08312 A1 discloses a tampon applicator in which the barrel has a larger diameter section for storing the tampon and a smaller diameter section which provides a finger grip portion.

Therefore, the present invention has been made in view of the above-described problem, and it is an object of the present invention to provide a tampon applicator which is capable of enhancing an effect of cleanly wiping menstrual blood adhering to a surface of an outer tube without need to employ an absorptive sheet for covering the outer tube.

Accordingly, a first aspect of the present invention consists in a tampon applicator comprising an outer tube for storing a tampon; and a push-out member which is inserted slidably into an opening portion at a rear end of the outer tube and is capable of compressing from the rear of the tampon that is stored in the outer tube and then pushing out the compressed tampon from an opening portion at a front end of the outer tube, wherein at least one opening is provided on an exterior face of the outer tube; characterized in that: the outer tube has a large-diameter section for storing the tampon and a small-diameter section which is smaller in diameter than the large-diameter section that is positioned at a base portion side more than the large-diameter section; the at least one opening is provided on an exterior face of the large-diameter section; and in the or at least one of the openings, a side face thereof at a front end side of the outer tube is adapted to tilt by a predetermined angle in an insertion direction of the tampon applicator and/or a side face thereof at a rear end side of the outer tube is adapted to tilt by a predetermined angle in a pullout direction of the tampon applicator.

A second aspect of the present invention consists in a tampon applicator comprising an outer tube for storing a tampon; and a push-out member which is inserted slidably into an opening portion at a rear end of the outer tube and is capable of compressing from the rear of the tampon that is stored in the outer tube and then pushing out the compressed tampon from an opening portion at a front end of the outer tube, wherein a plurality of convex portions are provided on an exterior face of the outer tube; characterised in that: the outer tube has a large-diameter section for storing the tampon and a small-diameter section which is smaller in diameter than the large-diameter section that is positioned at a base portion side more than the large-diameter section; the plurality of convex portions are provided on an exterior face of the large-diameter section; and in at least one of the convex portions, a height thereof at a rear end side of the outer tube is formed so as to be greater than a height thereof at a front end side of the outer tube.

As has been described above, according to the present invention, a tampon applicator can be provided which is capable of enhancing an effect of cleanly wiping menstrual blood adhering to a surface of an outer tube without a need to employ an absorptive sheet for covering the outer tube.

### [Brief Description of Drawings]

[Fig. 1] Fig. 1 is a perspective view of a tampon applicator according to a first embodiment of the present invention.
[Fig. 2] Fig. 2 is a sectional view taken along the line A-A' of the tampon applicator according to the first embodiment of the present invention.
[Fig. 3] Fig. 3 is an enlarged view of a large-diameter section in the sectional view taken along the line A-A' of the tampon applicator according to the first embodiment of the present invention.
[Fig. 4] Fig. 4 is a side view of a tampon applicator according to Exemplary Modification 1 of the present invention.
[Fig. 5] Fig. 5 is an enlarged view of a large-diameter section in the sectional view taken along the line A-A' of a tampon applicator according to Exemplary Modification 2 of the present invention.
[Fig. 6] Fig. 6 is a side view of a tampon applicator according to Exemplary Modification 3.
[Fig. 7] Fig. 7 is a perspective view of a tampon applicator according to a second embodiment of the present invention.
[Fig. 8] Fig. 8 is a sectional view taken along the line 100A-100A' of the tampon applicator according to the second embodiment of the present invention.
[Fig. 9] Fig. 9 is a sectional view taken along the line 100A-A' of the tampon applicator according to the second embodiment of the present invention.
[Fig. 10] Fig. 10 is a side view of the tampon applicator according to Exemplary Modification 1 of the present invention.
[Fig. 11] Fig. 11 is a side view of the tampon applicator according to Exemplary Modification 2 of the present invention.
[Fig. 12] Fig. 12 is an enlarged view of a large-diameter section in the sectional view taken along the line 100A-100A' of the tampon applicator according to Exemplary Modification 3 of the present invention.
[Fig. 13] Fig. 13 is an enlarged view of the large-diameter section in the sectional view taken along the line 100A-100A' of the tampon applicator according to Exemplary Modification 3 of the present invention.
[Fig. 14] Fig. 14 is a side view of a tampon applicator according to Exemplary Modification 4 of the present invention.
[Fig. 15] Fig. 15 is a perspective view of a tampon applicator according to a third embodiment of the present invention.
[Fig. 16] Fig. 16 is a sectional view taken along the line 1000A-1000A' of the tampon applicator according to the third embodiment of the present invention.
[Fig. 17] Fig. 17 is an enlarged view of a large-diameter section in the sectional view taken along the line 1000A-1000A' of the tampon applicator according to the third embodiment of the present invention.
[Fig. 18] Fig. 18 is a side view of the tampon applicator according to Exemplary Modification 1 of the present invention.
[Fig. 19] Fig. 19 is a side view of the tampon applicator according to Exemplary Modification 2 of the present invention.
[Fig. 20] Fig. 20 is a side view of the tampon applicator according to Exemplary Modification 3 of the present invention.
[Fig. 21] Fig. 21 is a side view of the tampon applicator according to Exemplary Modification 4 of the present invention.

### [Description of Embodiments]

### (Configuration of Tampon Applicator According to First Embodiment of the Present Invention)

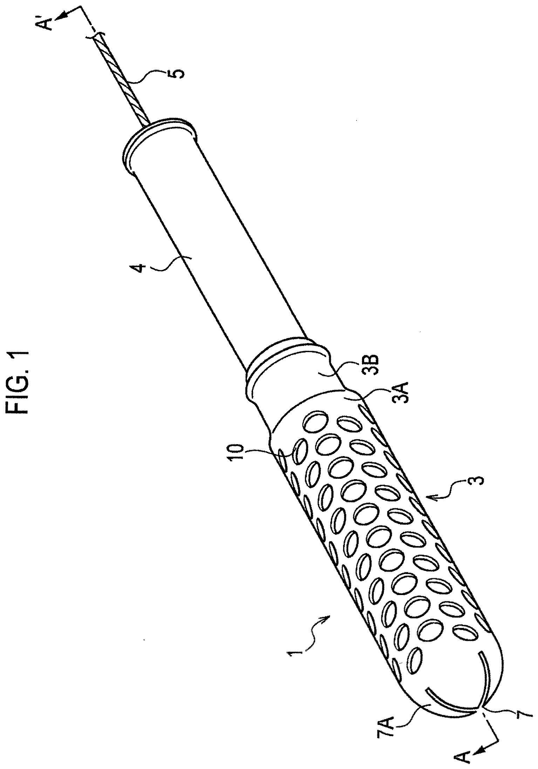

A configuration of a tampon applicator according to a first embodiment of the present invention will be described with reference to Fig. 1 to Fig. 3.

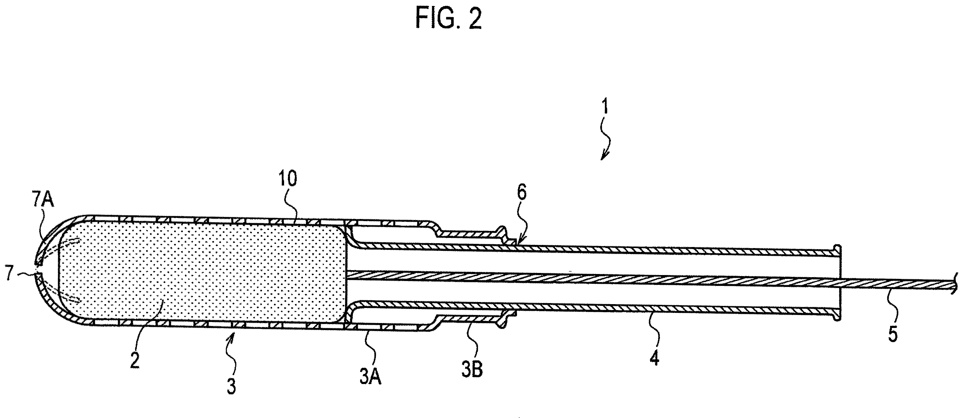

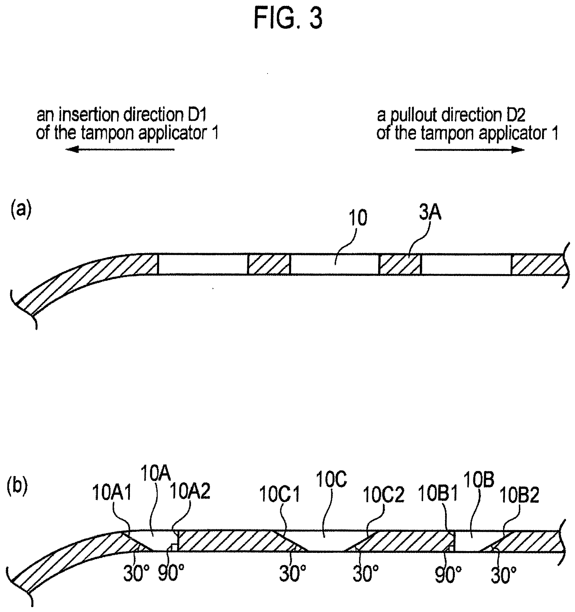

Fig. 1 is a perspective view of a tampon applicator 1 according to the embodiment; Fig. 2 is a sectional view taken along the line A-A' of the tampon applicator 1 according to the embodiment; and Fig. 3 (a) and Fig. 3 (b) are enlarged views of a large-diameter section 3A of an outer tube 3 in the sectional view taken along the line A-A' of the tampon applicator 1 according to the embodiment.

As shown in Fig. 1 and Fig. 2, the tampon applicator 1 according to the embodiment has an outer tube 3 and a push-out member 4.

The outer tube 3, a transverse cross section of which is formed in a substantially circular shape, has: a large-diameter section 3A for stored a tampon 2; and a small-diameter section 3B which is smaller in diameter than the large-diameter section 3A that is positioned at a base portion side more than the large-diameter section 3A.

At a front end of the outer tube 3, an opening portion 7 is formed by means of a radial slit. At the opening portion 7, a plurality of piece portions 7A is formed by means of such slit.

The push-out member 4 is slidably inserted into an opening portion 6 at a rear end of the outer tube 3 and is constituted to compress the tampon 2 stored in the large-diameter section 3A from a backward side so as to be thereby able to push out the compressed tampon from the opening portion 7 at the front end of the outer tube 3.

As shown in Fig. 2, in the tampon 2, a pullout cord 5 extends from a rear end of the tampon and such pullout cord 5 extends to the outside of the tampon applicator 1 through the inside of the push-out member 4.

In addition, the tampon 2 that is stored in the outer tube 3 is constituted to be pushed from a backward side by means of the push-out member 4 when it is inserted into virginal cavity and to push and widen the piece portions 7A that are formed at the opening portion 7 so as to be exposed to the outside of the outer tube 3.

In addition, at least one opening 10 is provided on an exterior face of the large-diameter section 3A. For example, as shown in Fig. 3(a) and Fig. 3 (b), the opening 10 that is provided on the exterior face of the large-diameter section 3A may be a plurality of through holes.

In an example of Fig. 3 (a), it is preferable that a diameter of the opening (through hole) 10 is 6 mm or less and that a thickness of the large-diameter section 3A is 1 mm or less.

In addition, in the example of Fig. 3 (a), it is preferable that intervals between the openings (through holes) 10 is 2 mm or more. Here, in a case where the intervals between the openings (through holes) 10 is 2 mm or more, it has a strength which is durable for a pressure at the time of transportation or use, and in a case where the intervals between the openings (through holes) 10 are 5 mm or more, it is stronger and it can impart a sense of easiness on appearance without exposing the tampon 2.

In addition, as shown in Fig. 3 (b), in a specific opening (through hole 10A, a side face 10A1 at a front end side of the outer tube 3 is adapted to tilt by a predetermined angle (for example, 30 degrees) in an insertion direction D1 of the tampon applicator 1.

In addition, in a specific opening (through hole) 10B, a side face 10B2 at a rear end side of the outer tube 3 is adapted to tilt by a predetermined angle (for example, 30 degrees) in a pullout direction D2 of the tampon applicator 1.

Further, in a specific opening (through hole) 10C, a side face 10C1 at the front end side of the outer tube 3 may be adapted to tilt by a predetermined angle (for example, 30 degrees) in the insertion direction D1 of the tampon applicator 1 and a side face 10C2 at the rear end side of the outer tube 3 may be adapted to tilt by a predetermined angle (for example, 30 degrees) in the pullout direction of the tampon applicator 1.

Moreover, it is preferable that the outer tube 3 is formed of a material of which surface energy is less than 37 × 10⁻³ N/m. The material of which surface energy is less than 37 × 10⁻³ N/m is assumed to include a material such as paper, rubber, or nylon which is surface-coated with the material of which surface energy is less than 37 × 10⁻³ N/m.

For example, the outer tube 3 is formed of: polyethylene of which surface energy is less than 31 × 10⁻³ N/m; polypropylene of which surface energy is less than 29 × 10⁻³ N/m; a silicon resin of which surface energy is less than 20 × 10⁻³ N/m; or polytetra fluoroethylene of which surface energy is less than 18.5 × 10⁻³ N/m or the like.

In a case where the outer tube 3 is formed of a material of which surface energy is 37 × 10⁻³ N/m or more (such as polyvinyl alcohol of which surface energy is 37 × 10⁻³ N/m or more, for example), the menstrual blood adhering to a surface of the outer tube 3 is absorbed into a material forming the outer tube 3; and therefore, use of the outer tube formed of such material is not preferable.

Here, surface energy can be analyzed by means of full automatic contact angle gauge DM700 which is available from Kyowa Interface Science Co., Ltd.

### (Functions and Advantageous Effect of Tampon Applicator According to First Embodiment of the Present Invention)

According to the tampon applicator 1 according to the first embodiment of the present invention, the applicator is constituted so that the menstrual blood having been adhered to the surface cylinder 3 in vaginal cavity is guided into a through hole 10, thus making it possible to avoid a circumstance that when the tampon applicator 1 is pulled out from the inside of vaginal cavity, a user's fingers are contaminated by the menstrual blood dropping along the surface of the outer tube 3.

In addition, with the tampon applicator 1 according to the first embodiment of the present invention, through holes 10 are provided all over an external face of the outer tube 3, thus making it possible to entirely reduce the menstrual blood dripping along the surface of the outer tube 3 when the tampon applicator 1 is pulled out from the inside of vaginal cavity.

In addition, with the tampon applicator 1 according to the first embodiment of the present invention, the applicator is constituted so that the menstrual blood that remains in the vaginal cavity when the tampon applicator 1 is inserted into vaginal cavity is guided and adheres to the through hole 10, thus making it possible to avoid initial use leakage from the tampon 2.

In particular, with the tampon applicator 1 according to the first embodiment of the present invention, since side faces of the through holes 10A to 10C tilt, when the tampon applicator 1 is inserted into vaginal cavity, the menstrual blood that remains in vaginal cavity can be guided to the through hole 10 more effectively.

Further, with the tampon applicator 1 according to the first embodiment of the present invention, even in a case where the surface of the outer tube 3 is wiped out by tissue paper or the like, menstrual blood still remains with the blood adhering to a side face of a through hole 10; and therefore, it is possible to readily discriminate whether or not the tampon applicator 1 has already been used.

### (Exemplary Modification 1)

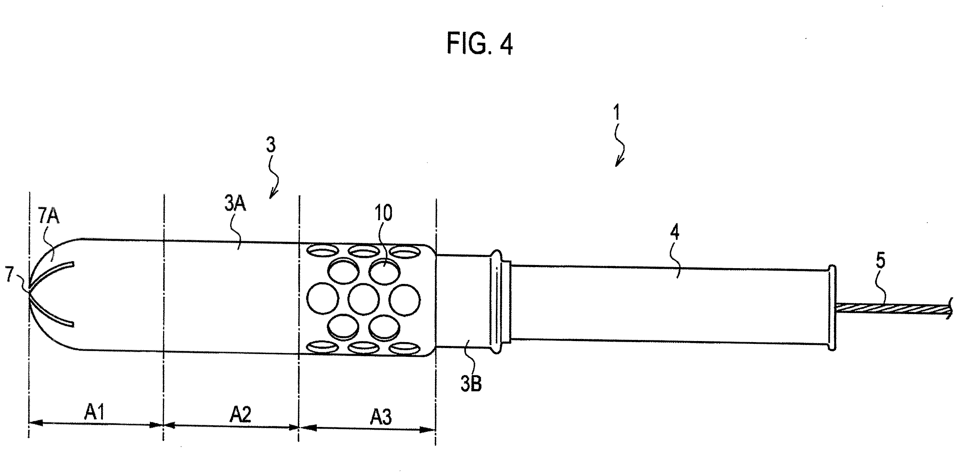

Referring to Fig. 4, a tampon applicator 1 according to Exemplary Modification 1 of the present invention will be described. Hereinafter, the tampon applicator 1 according to this Exemplary Modification 1 will be described focusing on differences from the tampon applicator 1 according to the first embodiment of the present invention.

In the tampon applicator 1 according to this Exemplary Modification 1, in a case where a large-diameter section 3A is equally divided into three portions, a forward portion A1, a central portion A2, and a backward portion A3, the number of openings 10 which are provided at the backward portion A3 is adapted to be more than that of openings 10 which are provided at the forward portion A1 and the central portion A2.

For example, as shown in Fig. 4, the abovementioned openings (for example, through holes) 10 may be provided at the backward portion A3 only. Such openings (for example, through holes) 10 may be provided at least at the backward portion A3, and it is preferable that the number of openings 10 which are provided at the backward portion A3 is more than that of openings 10 which are provided at the forward portion A1 and the central portion A2. With the tampon applicator 1 according to this Exemplary Modification 1, since the tampon applicator 1 is pulled out from the inside of vaginal cavity with the backward portion A3 down, the menstrual blood adhering to the tampon applicator 1 is likely to drip along the backward portion A3; and however, in a case where there are openings (through holes) 10 at the forward portion A1 or the central portion A2, there is a possibility that a hand or a cloth is contaminated due to running down of the menstrual blood adhering to the backward portion A3 side more than the openings (through holes) 10. Therefore, the menstrual blood that is running down can be reduced in a case of providing more openings 10 at the backward portion A3 more remarkably than in a case where more openings 10 are provided at the forward portion A1 and the central portion A2.

### (Exemplary Modification 2)

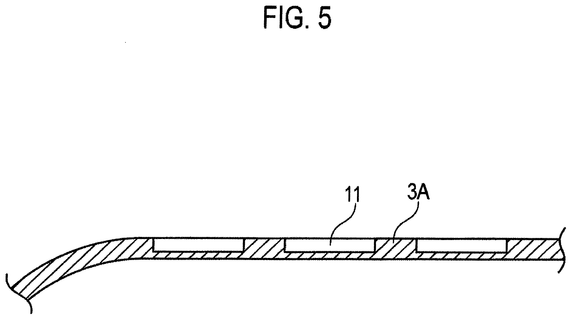

Referring to Fig. 5, a tampon applicator 1 according to Exemplary Modification 2 of the present invention will be described. Hereinafter, the tampon applicator 1 according to this Exemplary Modification 2 will be described focusing differences from the tampon applicator 1 according to the first embodiment of the present invention.

In the tampon applicator 1 according to Exemplary Modification 2 of the present invention, as shown in Fig. 5, an opening 11 which is provided on an exterior face of a large-diameter section 3A is formed by a hole having a predetermined depth instead of being formed by a through hole.

### (Exemplary Modification 3)

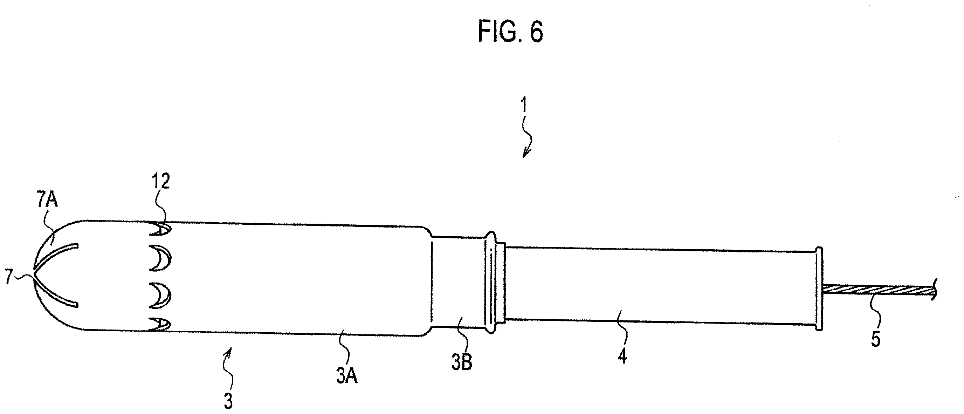

Referring to Fig. 6, a tampon applicator 1 according to Exemplary Modification 3 of the present invention will be described. Hereinafter, the tampon applicator 1 according to this Exemplary Modification 3 will be described focusing to differences from the tampon applicator 1 according to the first embodiment of the present invention.

In the tampon applicator 1 according to Exemplary Modification 2 of the present invention, an opening 11 which is provided on an exterior face of a large-diameter section 3A is formed in a noncircular shape. For example, as shown in Fig. 6, the abovementioned opening 12 may be formed in a shape which is curved to a rear end side of the outer tube 3 (in a crescent shape). In addition, the abovementioned opening 12 may be formed in a heart shape or in a character shape or the like.

### (Configuration of Tampon Applicator According to Second Embodiment of the Present Invention)

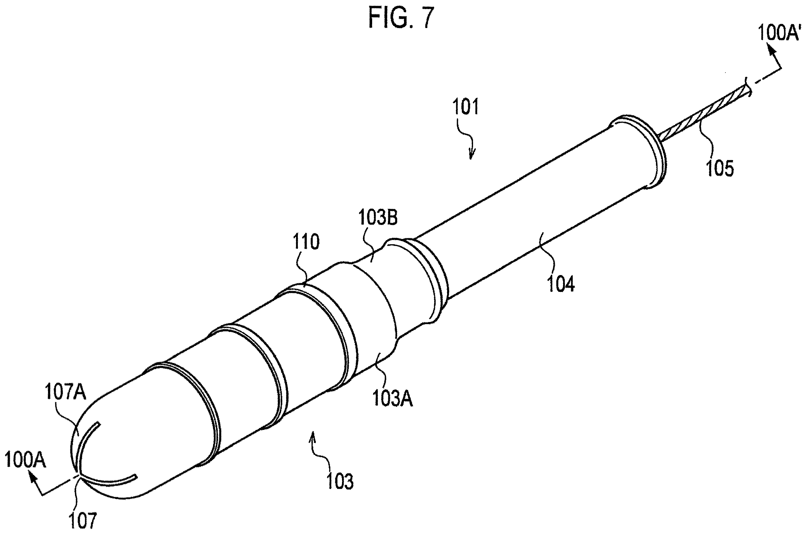

Referring to Fig. 7 to Fig. 9, a configuration of a tampon applicator according to a second embodiment of the present invention will be described.

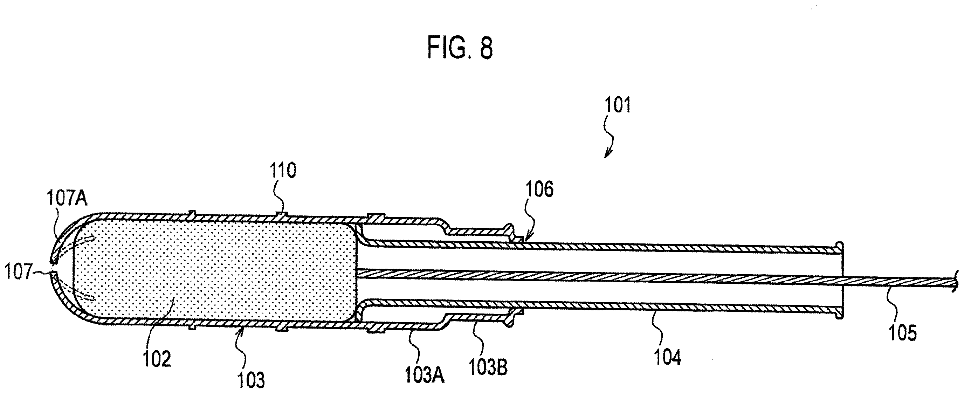

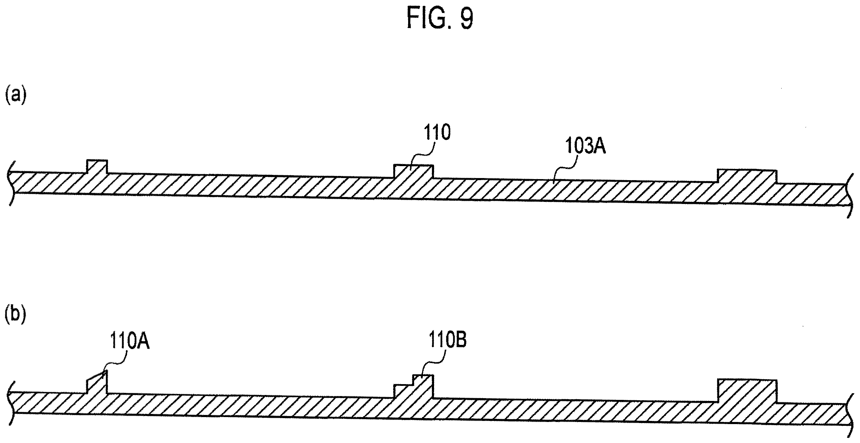

Fig. 7 is a perspective view of a tampon applicator 101 according to the embodiment; Fig. 8 is a sectional view taken along the line 100A-100A' of the tampon applicator 101 according to the embodiment; and Fig. 9(a) and Fig. 9 (b) are enlarged views of a large-diameter section 103A of an outer tube 103 in the sectional view taken along the line 100A-100A' of the tampon applicator 101 according to the embodiment.

As shown in Fig. 7 and Fig. 8, the tampon applicator 101 according to the embodiment has an outer tube 103 and a push-out member 104.

The outer tube 103, a transverse cross section of which is formed in a substantially circular shape, has: a large-diameter section 103A for storing a tampon 102; and a small-diameter section 103B of a smaller diameter than that of the large-diameter section 103A that is positioned at a base portion side more than the large-diameter section 103A.

At a front end of the outer tube 103, an opening portion 107 is formed by means of a radial slit. At such opening portion 107, a plurality of piece portions 107A is formed by means of such slit.

The push-out member 104 is inserted slidably into the opening portion 106 at a rear end of the outer tube 103 and is constituted to compress the tampon 102 that is stored in the large-diameter section 103A to be able to push out the compressed tampon from the opening diameter portion 107 at a front end of the outer tube 103.

As shown in Fig. 8, in the tampon 102, a pullout cord 105 extends from a rear end of the tampon, and such pullout cord 105 extends to the outside of the tampon applicator 101 through the inside of the push-out member 104.

In addition, the tampon 102 that is stored in the outer tube 103 is pushed from a backward side by means of the push-out member 104 when it is inserted into vaginal cavity to push and widen the piece portions 107A that are formed at the opening portion 107 so as to be exposed to the outside of the outer tube 103.

In addition, a plurality of convex portions 110 are provided on an exterior face of the large-diameter section 103A. As shown in Fig. 7, at least one of the convex portions 110 is formed in a continuous shape and is formed so as to go around the large-diameter section 103A.

For example, as shown in Fig. 7, a plurality of convex portions (convex grooves) 110 that are formed in the continuous shape and are formed so as to go around the large-diameter section 103A may be provided in parallel to each other. Such convex portions 110 may be constituted so as to go around in a cross section which is vertically cut in a longitudinal direction of the tampon applicator 101 or may be constituted so as to go around in the other cross section.

In addition, a plurality of convex portions 110 may be formed along an outer circumference of the large-diameter section 103A. In addition, the convex portions 110 may be formed in a continuous shape and may be formed in part of the large-diameter section 103A.

In an example of Fig. 9 (a), it is preferable that a height of the convex portion 110 is 0.1 mm or more and 3 mm or less. Here, in a case where the height of the convex portion 110 is 3 mm or less, a rugged sense of feeling on appearance or a sense of discomfort at the time of use can be avoided. Further, in a case where the height of the convex portion 110 is 1.5 mm or less, even if the convex portion 110 touches a user's skin, the user does not have a feeling of discomfort such as a pain and can insert the tampon applicator 101 into her vaginal cavity. If the height of the convex portion 110 is 0.1 mm or more, an effect of wiping out the menstrual blood adhering to a surface of the outer tube 103 cannot be obtained.

In the example of Fig. 9 (a), it is preferable that intervals between the convex portions 110 are 1 mm or more. Here, if the intervals between the convex portions 110 are less than 1 mm, there is a possibility that, when the outer tube 103 is removed from a die, the convex portion 110 breaks or has a chip. In addition, if the intervals between the convex portions 110 are too narrow, since the capacitance of the menstrual blood entering between the convex portion 110 and the convex portion 110 lessens and the menstrual blood overflows without adhering to a side face of the convex portion 110, it is preferable that the intervals between the convex portions 110 are 5 mm or more.

In addition, widths of the plurality of convex portions 110 may be identical to or may be different from each other. It is preferable that the intervals of the convex portions 110 each are 0.1 mm or more and 8 mm or less. Here, in a case where the widths of the convex portions 110 each are less than 0.1 mm, there is a possibility that a user feels a pain or damages the inside of vaginal cavity at the time of using the tampon applicator 101 and there is a possibility that when the outer tube 103 is removed from a die, the convex portion 110 breaks or has a chip.

On the other hand, if the widths of the convex portions 110 each are 0.4 mm or more and 3 mm or less, a user can use the tampon applicator 101 without any pain and a manufacturer can stably manufacture the tampon applicator 101. If the widths of the convex portions 110 each are greater than 8 mm, an effect of wiping out the menstrual blood adhering to the surface of the outer tube 103 cannot be obtained.

Further, in a specific convex portion 110, at least either one of a side face at a front end side of the outer tube 103 and a side face at a rear end side of the outer tube 103 may be constituted so as to tilt by a predetermined angle in an insertion direction of the tampon applicator 101 or in a pullout direction of the tampon applicator 101.

Moreover, in at least one of the convex portions 110, a height of a rear end side of the outer tube 103 may be formed so as to be greater than that of a front end side of the outer tube 103.

For example, as shown in Fig. 9 (b), a top face of the convex portion 110A may tilt so that the height of the rear end side of the outer tube 103 is greater than that of the front end side of the outer tube 103 or a step difference may be provided on the top face of the convex portion 110B.

In addition, it is preferable that the outer tube 103 is formed of a material of which surface energy is less than 37 x 10⁻³ N/m. The material of which surface energy is less than 37 x 10⁻³ N/m is assumed to include a material such as paper, rubber, or nylon which is surface-coated with the material of which surface energy is less than 37 x 10⁻³ N/m.

For example, the outer tube 103 is formed of: polyethylene of which surface energy is less than 31 × 10⁻³ N/m; polypropylene of which surface energy is less than 29 × 10⁻³ N/m; a silicone resin of which surface energy is less than 20 × 10⁻³ N/m; or polytetra fluoroethylene of which surface energy is less than 18.5 × 10⁻³ N/m or the like.

In a case where the outer tube 103 is formed of a material of which surface energy is 37 × 10⁻³ N/m or more (such as polyvinyl alcohol of which surface energy is 37 × 10⁻³ N/m or more, for example), the menstrual blood adhering to a surface of the outer tube 103 is absorbed into a material forming the outer tube 103; and therefore, use of the outer tube formed of such material is not preferable.

Here, surface energy can be analyzed by means of full automatic contact angle gauge DM700 which is available from Kyowa Interface Science Co., Ltd.

### (Functions and Advantageous Effects of Tampon Applicator According to Second Embodiment of the Present Invention)

With a tampon applicator 101 according to the second embodiment, the menstrual blood having been adhered to a surface of an outer tube 103 in vaginal cavity is adapted to adhere to a side face of a convex portion 110, thus making it possible to avoid a circumstance that when the tampon applicator 101 is pulled out from the inside of vaginal cavity, a user's fingers are contaminated by the menstrual blood running down along a surface of the outer tube 103.

In addition, with the tampon applicator 101 according to the second embodiment of the present invention, since a convex portion 110 which is formed in a continuous shape so as to go around an exterior face of the outer tube 103 is provided, the menstrual blood running down along the surface of the outer tube 103 when the tampon applicator 101 is pulled out from the inside of vaginal cavity can be prevented.

In addition, with the tampon applicator 101 according to the second embodiment of the present invention, since the top face of the convex portion 110A, 110B tilts, the menstrual blood that remains in vaginal cavity when the tampon applicator 101 is inserted into vaginal cavity can be adhered to the side face of the convex portion 110A, 110B more effectively.

Further, with the tampon applicator 101 according to the second embodiment of the present invention, even in a case where the surface of the outer tube 103 has been wiped out by means of tissue paper or the like, since the menstrual blood remains with it adhering to the side face of the convex portion 110, it is possible to readily discriminate whether or not the tampon applicator 101 is the one that has already been used.

### (Examplary Modification 1)

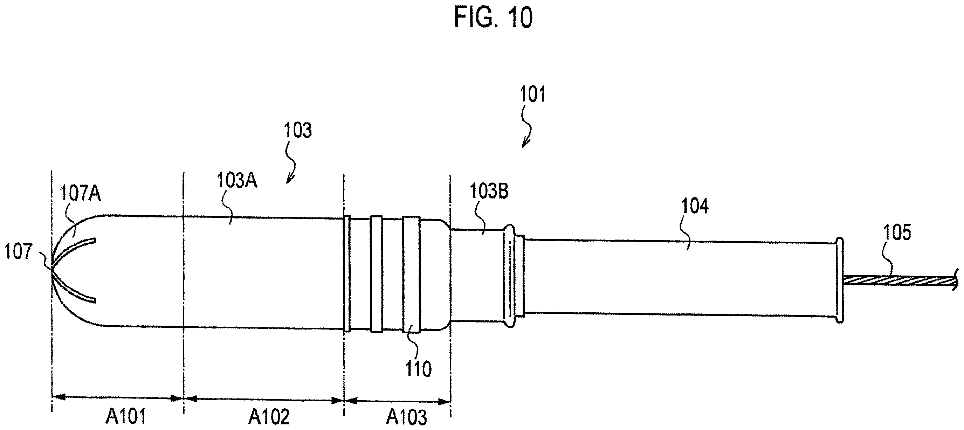

Referring to Fig. 10, a tampon applicator 101 according to Exemplary Modification 1 of the present invention will be described. Hereinafter, the tampon applicator 101 according to this Exemplary Modification 1 will be described focusing on differences from the tampon applicator 101 according to the second embodiment of the present invention.

In the tampon applicator 101 according to this Exemplary Modification 1, in a case where a large-diameter section 103A is equally divided into three sections, a forward portion A101, a central portion A102, and a backward portion A103, the applicator is constituted so that the number of convex portions 110 which are provided at the backward portion A103 is more than that of convex portions 110 which are provided at the forward portion A101 and the central portion A102.

For example, as shown in Fig. 10, the abovementioned convex portions 110 may be provided at the backward portion A103 only. The convex potions 110 may be provided at least at the backward portion A103, and the number of convex portions 110 which are provided at the backward portion A103 may be greater than that of convex portions 110 which are provided at the forward portion A101 and the central portion A102. With the tampon applicator 101 according to Exemplary Modification 1, since the tampon applicator 101 is pulled out from the inside of vaginal cavity with the backward portion A103 side down, the menstrual blood adhering to the tampon applicator 101 is likely to run along the backward portion A103; and however, in a case where a convex portion 110 is present at the forward portion A101 or the central portion A102, there is a possibility that a hand or a cloth is contaminated due to running down of the menstrual blood adhering to the backward portion A103 side more than the convex portion 110. Therefore, the menstrual blood that is running down can be reduced in a case of providing more convex portions 110 at the backward portion A103 more remarkably than in a case where more convex portions 110 are provided at the forward portion A101 and the central portion A102.

### (Exemplary Modification 2)

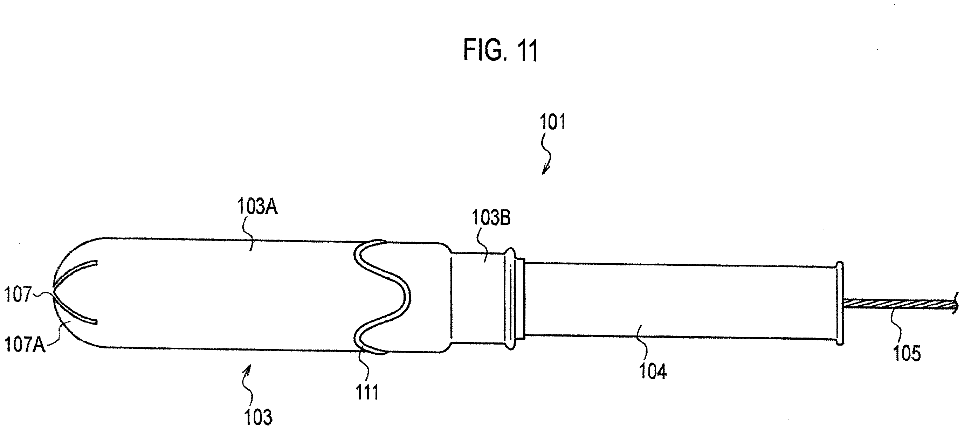

Referring to Fig. 11, a tampon applicator 101 according to Exemplary Modification 2 of the present invention will be described. Hereinafter, the tampon applicator 101 according to Exemplary Modification 2 will be described focusing on differences from the tampon applicator 101 according to the second embodiment of the present invention.

As shown in Fig. 11, in the tampon applicator 101 according to this Exemplary Modification 2, at least one of convex portions 111 which are provided on an outer face of an outer tube 103 is formed in a continuous, substantially wave-like shape and is formed so as to go around a large-diameter section 103A.

### (Exemplary Modification 3)

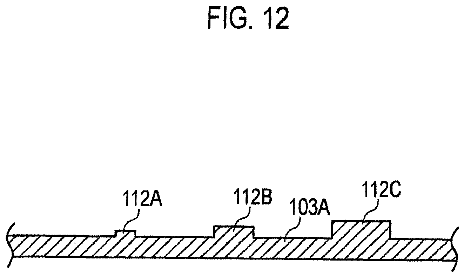

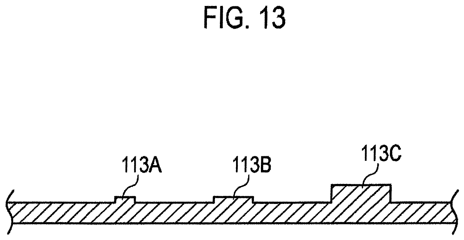

Referring to Fig. 12 and Fig. 13, a tampon applicator 101 according to Exemplary Modification 3 of the present invention will be described. Hereinafter, the tampon applicator 101 according to this Exemplary Modification 3 will be described focusing on the tampon applicator 101 according to the second embodiment of the present invention.

Here, in the tampon applicator 101 according to this Exemplary Modification 3, a height of a convex portion which is provided at the rearmost end side of an outer tube 103 is formed so as to be the greatest among a plurality of convex portions.

For example, as shown in Fig. 12, a height of a convex portion 112C which is provided at the rear end side of the outer tube 103 which is provided at the most outer part is formed so as to be the greatest among a plurality of convex potions 112A to 112C. Here, a height of the convex portion 112A may be smaller than that of a convex portion 112B which is provided at a rear end side of the outer tube 103 more than the convex portion 112A.

In addition, as shown in Fig. 13, the height of the convex portion 113C that is provided at the most rear end side of the outer tube 103 is formed so as to be the greatest among the plurality of convex potions 113A to 113C. Here, the heights of the convex portions 113A, 113B other than the convex portion 113C may be identical to each other.

### (Exemplary Modification 4)

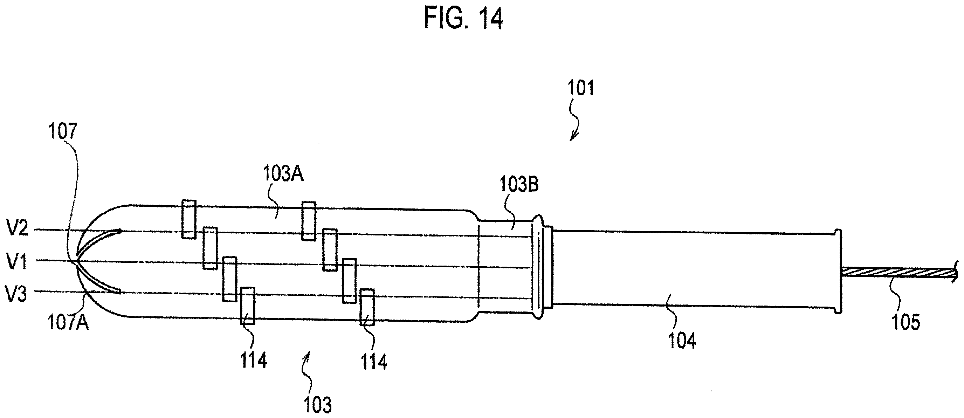

Referring to Fig. 14, a tampon applicator 101 according to Exemplary Modification 4 of the present invention will be described. Hereinafter, the tampon applicator 101 according to this Exemplary Modification 4 will be described focusing on differences from the tampon applicator 101 according to the second embodiment of the present invention.

Here, in the tampon applicator 101 according to this Exemplary Modification 4, at least one of convex portions 114 which are provided at an outer tube 103 is formed in an intermittent shape and is formed so as to go around a large-diameter section 103A.

Specifically, as shown in Fig. 14, in the tampon applicator 101 according to this Exemplary Modification 4, in a case where imaginary lines V1 to V3 are drawn along a longitudinal direction, the imaginary lines V1 to V3 are adapted to always cross the convex potions 114 that are formed so as to go around the large-diameter section 103A in an intermittent shape.

### (Configuration of Tampon Applicator According to Third Embodiment of the Present Invention)

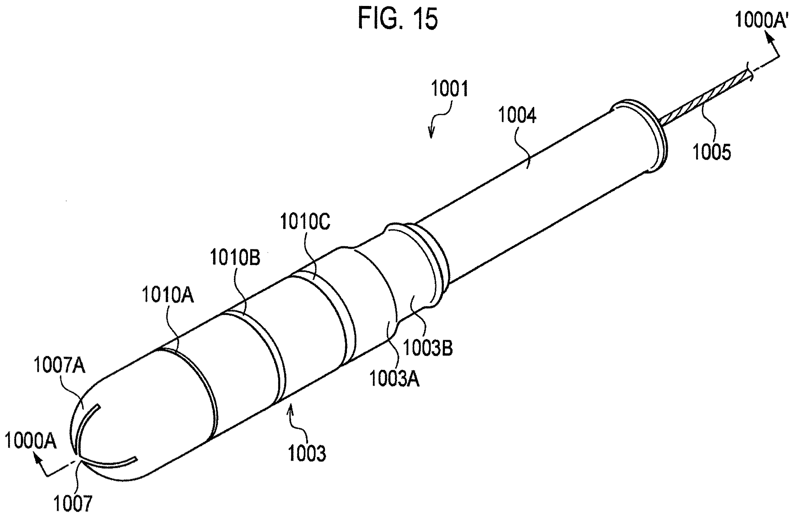

Referring to Fig. 15 to Fig. 17, a configuration of a tampon applicator according to a third embodiment of the present invention will be described.

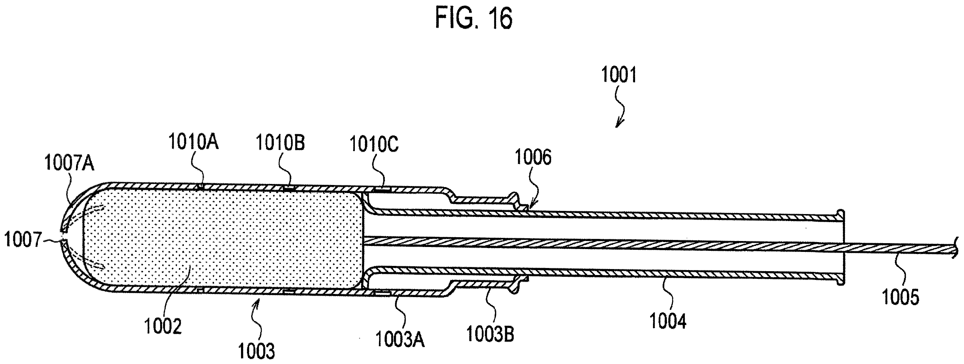

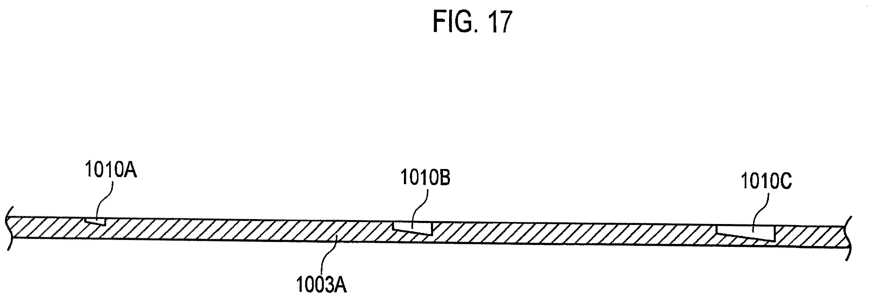

Fig. 15 is a perspective view of a tampon applicator 1001 according to the embodiment; Fig. 16 is a sectional view taken along the line 1000A-1000A' of the tampon applicator 1001 according to the embodiment; and Fig. 17 is an enlarged view of a large-diameter section 1003A of an outer tube 1003 in the sectional view taken along the line 1000A-1000A' of the tampon applicator 1001 according to the embodiment.

As shown in Fig. 15 and Fig. 16, the tampon applicator 1001 according to the embodiment has an outer tube 1003 and a push-out member 1004.

The outer tube 1003, a transverse cross section of which is formed in a substantially circular shape, has: a large-diameter section 1003A for storing a tampon 1002; and a small-diameter section 1003B which is smaller in diameter than the larger diameter 1003A that is positioned at a base portion side more than the large-diameter section 1003A.

At a front end of the outer tube 1003, an opening portion 1007 is formed by means of a radial slit. At such opening portion 1007, a plurality of piece portions 1007A are formed by such slit.

The push-out member 1004 is inserted slidably into an opening portion 1006 at a rear end of the outer tube 1003 and is constituted to compress the tampon 1002 that is stored in the large-diameter section 1003A so as to be thereby able to push out the compressed tampon from the opening portion 1007 at a front end of the outer tube 1003.

As shown in Fig. 16, in the tampon 1002, a pullout cord 5 extends from a rear end of the tampon and such pullout cord 5 extends to the outside of the tampon applicator 1001 through the inside of a push-out member 1004.

In addition, the tampon 1002 that is stored in the outer tube 1003 is constituted to be pushed from a backward side by means of the push-out member 1004 when it is inserted into vaginal cavity and to push and open the piece portions 1007A that are formed at the opening portion 1007 so as to be exposed to the outside of the outer tube 1003.

In addition, a plurality of concave portions 1010A to 1010C are provided at an exterior face of the large-diameter section 1003A. As shown in Fig. 15, at least one of the concave portions 1010A to 1010C is formed in a continuous shape and is formed so as to go around the large-diameter section 1003A.

For example, as shown in Fig. 15, a plurality of concave portions (concave grooves) 1010A to 1010C that are formed in a continuous shape and are formed so as to go around the large-diameter section 1003A may be provided in parallel to each other. Such concave grooves 1010A to 1010C may be constituted so as to go around in a cross section which is vertically cut in a longitudinal direction of the tampon applicator 1001 or may be constituted so as to go around in the other cross section.

In addition, a plurality of concave portions 1010A to 1010C may be formed along an outer circumference of the large-diameter section 1003A. In addition, the concave portions 1010A to 1010C may be formed in a continuous shape and is formed in part of the large-diameter section 1003A.

As shown in Fig. 17, in the concave portions 1010A to 1010C, the depth of a rear end side of the outer tube 1003 may be formed so as to be greater than that of a front end side of the outer tube 1003.

In addition, as shown in Fig. 17, a depth of the concave portion 1010C that is provided at the rearmost end side of the outer tube 1003 (the depth of the deepest part in the concave portion 1010C) may be formed so as to be the greatest among the plurality of concave portions 1010A to 1010C.

Here, a depth of the concave portion 1010A (the depth of the deepest part in the concave portion 1010A) may be smaller than a depth of the concave portion 1010B that is provided at a rear end side of the outer tube 1003 more than the concave portion 1010A (the depth at the deepest part in the concave portion 1010B) or depths of the concave portions 1010A, 1010B other than the concave portion 1010C (the depth of the deepest part in the concave portions 1010A, 1010B) may be identical to each other.

In addition, the widths of the plurality of concave portions 1010A to 1010C may be identical to or different from each other. Further, it is preferable that intervals among the concave portions 1010A to 1010C are 5 mm or more.

In addition, in specific concave portions 1010A to 1010C, at least one of a side face at the front end side of the outer tube 1003 and a side face at the rear end side of the outer tube 1003 may be constituted so as to tilt by a predetermined angle in an insertion direction of the tampon applicator 1001 or in a pullout direction of the tampon applicator 1001.

Moreover, it is preferable that the outer tube 1003 is formed of a material of which surface energy is less than 37 × 10⁻³ N/m. The material of which surface energy is less than 37 × 10⁻³ N/m is assumed to include a material such as paper, rubber, or nylon which is surface-coated with the material of which surface energy is less than 37 × 10⁻³ N/m.

For example, the outer tube 1003 is formed of: polyethylene of which surface energy is less than 31 × 10⁻³ N/m; polypropylene of which surface energy is less than 29 × 10⁻³ N/m; a silicon resin of which surface energy is less than 20 × 10⁻³ N/m; or polytetra fluoroethylene of which surface energy is less than 18.5 × 10⁻³ N/m or the like.

In a case where the outer tube 1003 is formed of a material of which surface energy is 37 × 10⁻³ N/m or more (such as polyvinyl alcohol of which surface energy is 37 × 10⁻³ N/m or more, for example), the menstrual blood adhering to a surface of the outer tube 1003 is absorbed into a material forming the outer tube 1003; and therefore, use of the outer tube formed of such material is not preferable.

Here, surface energy can be analyzed by means of full automatic contact angle gauge DM700 which is available from Kyowa Interface Science Co., Ltd.

### (Functions and Advantageous Effects of Tampon Applicator According to Third Embodiment of the Present Invention)

With a tampon applicator 1001 according to the third embodiment of the present invention, the menstrual blood having been adhered to a surface of an outer tube 1003 in vaginal cavity is adapted to be guided into concave portions 1010A to 1010C, thus making it possible to avoid a circumstance that a user's fingers are contaminated due to the menstrual blood running down along the surface of the outer tube 1003 when the tampon applicator 1001 is pulsed out from the inside of vaginal cavity.

In addition, with the tampon applicator 1001 according to the third embodiment of the present invention, concave portions (concave grooves) 1010A to 1010C are provided in a continuous shape so as to go around an exterior face of the outer tube 1003, thus making it possible to entirely reduce the menstrual blood running down along the surface of the outer tube 1003 when the tampon applicator 1001 is pulled out from the inside of vaginal cavity.

In addition, with the tampon applicator 1001 according to the third embodiment of the present invention, since the side faces of the concave portions 1010A to 1010C tilt, the menstrual blood that remains in vaginal cavity when the tampon applicator 1001 is inserted into vaginal cavity can be guided into the concave portions 1010A to 1010C more effectively.

Further, with the tampon applicator 1001 according to the third embodiment of the present invention, even in a case where the surface of the outer tube 1003 has been wiped out by means of tissue paper or the like, since the menstrual blood remains with the blood adhering to the side faces of the concave potions 1010A to 1010C, it is possible to readily discriminate whether or not such tampon applicator 1001 is the one that has been already used.

### (Exemplary Modification 1)

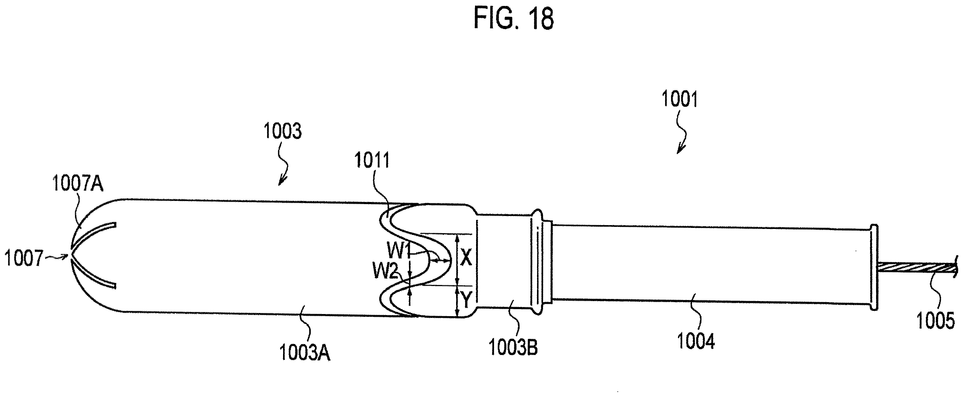

Referring to Fig. 18, a tampon applicator 1001 according to Exemplary Modification 1 of the present invention will be described. Hereinafter, the tampon applicator 1001 according to this Exemplary Modification 1 will be described focusing on differences from the tampon applicator 1001 according to the third embodiment of the present invention.

As shown in Fig. 18, in the tampon applicator 1001 according to this Exemplary Modification 2, at least one of concave portions 1011 that are provided on an exterior face of an outer tube 1003 is formed in a continuous, substantially wave-like shape, and is formed so as to go around a large-diameter section 1003A.

Here, in the concave portion 1011, portions with their different widths are provided. For example, as shown in Fig. 18, in the concave portion 1011, a width W1 of a valley-shaped portion X which is curved to a rear end side of the outer tube 1003 may be formed to be greater than a width W2 of a mountain-shaped portion Y which is curved to a front end side of the outer tube 1003.

In addition, as shown in Fig. 18, in the concave portion 1011, a depth of the valley-shaped portion that is curved to the rear end side of the outer tube 1003 may be formed to be greater than that of the mountain-shaped portion Y that is curved to the front end side of the outer tube 1003.

### (Exemplary Modification 2)

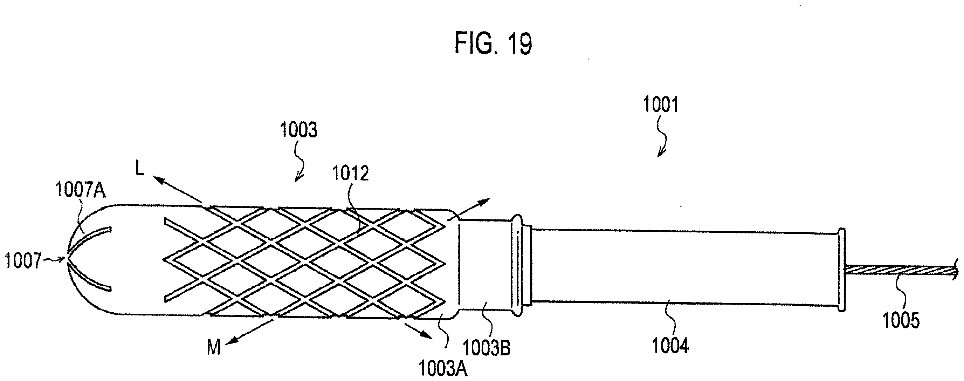

Referring to Fig. 19, a tampon applicator 1001 according to Exemplary Modification 2 of the present invention will be described. Hereinafter, the tampon applicator 1001 according to this Exemplary Modification 2 will be described focusing on differences from the tampon applicator 1001 according to the third embodiment of the present invention.

As shown in Fig. 19, a plurality of concave portions (concave grooves) 1012 which are formed in a continuous shape are formed so as to go around in a cross section which is parallel to two planes L and M which cross each other. A plane L and a plane M may be orthogonal to each other or may cross each other at a predetermined angle.

### (Exemplary Modification 3)

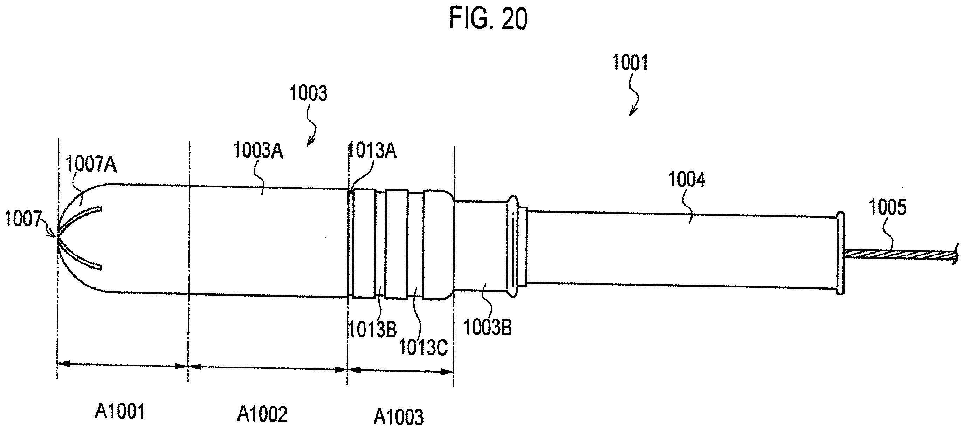

Referring to Fig. 20, a tampon applicator 1001 according to Exemplary Modification 3 of the present invention will be described. Hereinafter, the tampon applicator 1001 according to this Exemplary Modification 3 will be described focusing on differences from the tampon applicator 1001 according to the third embodiment of the present invention.

In the tampon applicator 1001 according to this Exemplary Modification 3, in a case where a large-diameter section 1003A is equally divided into three portions, a forward portion A1001, a central portion A1002, and a backward portion A1003, the number of concave portions 1013A to 1013C which are provided at the backward portion A1003 is constituted so as to be greater than that of concave portions which are provided at the forward portion A1001 and the central portion A1002.

For example, as shown in Fig. 20, the abovementioned concave portions 1013A to 1013C may be provided at the backward portion A1003 only.

For example, as shown in Fig. 20, the abovementioned concave portions 1013A to 1013C may be provided at the backward portion A1003 only. Such concave portions 1013A to 1013C may be provided at least at the backward portion A1003, and it is preferable that the number of concave portions 1013 which are provided at the backward portion A1003 is more than that of openings 13 which are provided at the forward portion A1001 and the central portion A1002.

With the tampon applicator 1001 according to this Exemplary Modification 3, since the tampon applicator 1001 is pulled out from the inside of vaginal cavity with the backward portion A1003 side down, the menstrual blood adhering to the tampon applicator 1001 is likely to run down along the backward portion A1003; and however, in a case where concave portions 1013A to 1013C are provided at the forward portion A1001 or the central portion A1002, there is a possibility that a hand or a cloth is contaminated due to running down of the menstrual blood adhering to the backward portion A1003 side more than such concave portions 1013A to 1013C. Therefore, the menstrual blood that is running down can be reduced in a case of providing more concave portions 1013 at the backward portion A1003 more remarkably in a case where more concave portions 1013 are provided at the forward portion A1001 and the central portion A1002.

With the tampon applicator 1001 according to this Exemplary Modification 3, the menstrual blood that is running down along the surface of the outer tube 1003 when the tampon applicator 1001 is pulled out from the inside of vaginal cavity can be reduced more remarkably than in the case where more concave portions 1013A to 1013C are provided at the forward portion A1001 and the central portion A1002.

### (Exemplary Modification 4)

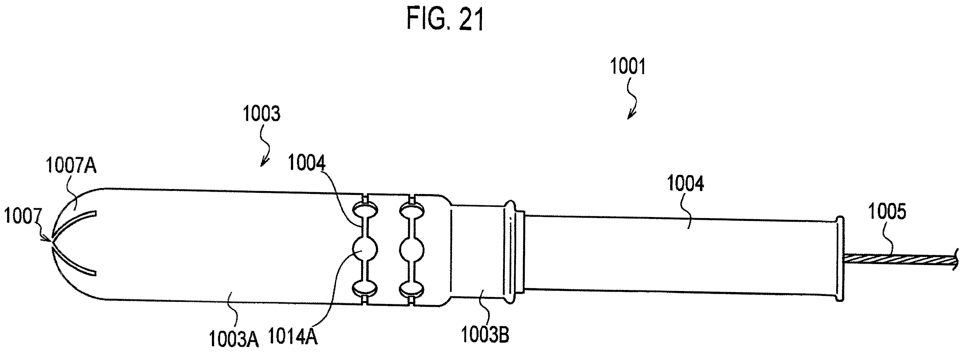

Referring to Fig. 21, a tampon applicator 1001 according to Exemplary Modification 4 of the present invention will be described. Hereinafter, the tampon applicator 1001 according to this Exemplary Modification 4 will be described focusing on differences from the tampon applicator 1001 according to the third embodiment of the present invention.

As shown in Fig. 21, an opening (through hole or non-through hole) 1014A may be provided on a concave portion (concave groove) 1014 which is formed in a continuous shape and is formed so as to go around an exterior face of an outer tube 1003. A diameter of such opening 1014A may be formed so as to be greater than a width of a concave portion 14.

While the present invention has been described in detail by employing the foregoing embodiments, it is apparent that the present invention is not limitative to the embodiments described in the specification. The present invention can be implemented as alteration and modification aspects without deviating from the gist and scope of the present invention that is defined by the recitations of the claims that follow. Therefore, the descriptive matters of the specification are merely intended to furnish illustrative explanation and do not have any limitative meaning to the present invention.

As has been described above, according to the present invention, a tampon applicator can be provided which is capable of enhancing an effect of cleanly wiping menstrual blood adhering to a surface of an outer tube without a need to employ an absorptive sheet for covering the outer tube. The present invention is thus advantageous in a tampon applicator.

## Claims

1. A tampon applicator (1) comprising:
an outer tube (3) for storing a tampon (2); and
a push-out member (4) which is inserted slidably into an opening portion (6) at a rear end of the outer tube (3) and is capable of compressing from the rear of the tampon (2) that is stored in the outer tube (3) and then pushing out the compressed tampon (2) from an opening portion (7) at a front end of the outer tube (3),
wherein at least one opening (10) is provided on an exterior face of the outer tube (3);
**characterized in that**:
the outer tube (3) has a large-diameter section (3A) for storing the tampon (2) and a small-diameter section (3B) which is smaller in diameter than the large-diameter section (3A) that is positioned at a base portion side more than the large-diameter section (3A);
the at least one opening (10) is provided on an exterior face of the large-diameter section (3A); and
in the or at least one (10A, 10B, 10C) of the openings (10), a side face (10A1, 10C1) thereof at a front end side of the outer tube (3) is adapted to tilt by a predetermined angle in an insertion direction (D1) of the tampon applicator (1) and/or a side face (10B2, 10C2) thereof at a rear end side of the outer tube (3) is adapted to tilt by a predetermined angle in a pullout direction (D2) of the tampon applicator (1).

2. The tampon applicator according to claim 1, wherein the opening (10) is a through hole.

3. The tampon applicator according to claim 1 or 2, wherein, in a case where the large-diameter section (3B) is equally divided into three portions, a forward portion (A1), a central portion (A2), and a backward portion (A3), the number of openings (10) which are provided at the backward portion (A3) is more than the number of openings (10) which are provided at the forward portion (A1) and the central portion (A2).

4. The tampon applicator according to claim 1, 2 or 3, wherein a diameter of the opening (10) is 6 mm or less and a thickness of the large-diameter section (3A) is 1 mm or less.

5. The tampon applicator according to any one of claims 1 to 4, wherein intervals of the openings (10) are 2 mm or more.

6. A tampon applicator (10) comprising:
an outer tube (103) for storing a tampon (102); and
a push-out member (104) which is inserted slidably into an opening portion (106) at a rear end of the outer tube (103) and is capable of compressing from the rear of the tampon (102) that is stored in the outer tube (103) and then pushing out the compressed tampon (102) from an opening portion (107) at a front end of the outer tube (103),
wherein a plurality of convex portions (110) are provided on an exterior face of the outer tube (103);
**characterised in that**:
the outer tube (103) has a large-diameter section (103A) for storing the tampon (102) and a small-diameter section (103B) which is smaller in diameter than the large-diameter section (103A) that is positioned at a base portion side more than the large-diameter section (103A);
the plurality of convex portions (110) are provided on an exterior face of the large-diameter section (103A); and
in at least one (110A, 110B) of the convex portions (110), a height thereof at a rear end side of the outer tube (103) is formed so as to be greater than a height thereof at a front end side of the outer tube (103).

7. The tampon applicator according to claim 6, wherein, in a case where the large-diameter section (103A) is equally divided into three portions, a forward portion (A101), a central portion (A102), and a backward portion (A103), the number of convex portions (110) which are provided at the backward portion (A103) is more than the number of convex portions (110), which are provided at the forward portion (A101) and the central portion (A102).

8. The tampon applicator according to claim 6 or 7, wherein at least one (111) of the convex portions (110) is formed in a continuous or intermittent shape and is formed so as to go around the large-diameter section (103A).

9. The tampon applicator according to claim 8, wherein at least one (111) of the convex portions (110) is formed in a substantially wave-like shape.

10. The tampon applicator according to any one of claims 6 to 9, wherein a height of a convex portion (112C, 113C) which is provided at a rearmost end side of the outer tube (103) is formed so as to be the greatest among the plurality of convex portions (112A, 112B, 112C; 113A, 113B, 113C).

11. The tampon applicator according to any one of claims 6 to 10, wherein the height of the convex portions (110) is 0.1 mm or more and 3 mm or less.

12. The tampon applicator according to any one of claims 6 to 11, wherein intervals between the convex portions (110) are 1 mm or more.

13. The tampon applicator according to any preceding claim, wherein the outer tube (3; 103) is formed of a material of which surface energy is less than 37 x 10⁻³ N/m.

## Patentansprüche

1. Tampon-Applikator (1), umfassend:
ein äußeres Rohr (3) zum Aufbewahren eines Tampons (2); und
ein Herausdrückelement (4), das verschiebbar in einen Öffnungsteil (6) an einem hinteren Ende des äußeren Rohrs (3) eingeführt wird und dazu fähig ist, von der Rückseite des Tampons (2) aus, der in dem äußeren Rohr (3) aufbewahrt ist, diesen zusammenzudrücken und dann den zusammengedrückten Tampon (2) aus einem Öffnungsteil (7) an einem vorderen Ende des äußeren Rohrs (3) herauszudrücken,
wobei mindestens eine Öffnung (10) an einer Außenfläche des äußeren Rohrs (3) vorgesehen ist;
**dadurch gekennzeichnet, dass**:
das äußere Rohr (3) einen Abschnitt (3A) mit großem Durchmesser zum Aufbewahren des Tampons (2) und einen Abschnitt (3B) mit kleinem Durchmesser hat, dessen Durchmesser kleiner als derjenige des Abschnitts (3A) mit großem Durchmesser ist, der mehr an einer Basisteilseite als der Abschnitt (3A) mit großem Durchmesser angeordnet ist;
die mindestens eine Öffnung (10) an einer Außenfläche des Abschnitts (3A) mit großem Durchmesser vorgesehen ist; und
bei der oder bei mindestens einer (10A, 10B, 10C) der Öffnungen (10) eine Seitenfläche (10A1, 10C1) davon an einer vorderen Endseite des äußeren Rohrs (3) dazu angepasst ist, in einem vorbestimmten Winkel in einer Einführrichtung (D1) des Tampon-Applikators (1) geneigt zu sein, und/oder eine Seitenfläche (10B2, 10C2) davon an einer hinteren Endseite des äußeren Rohrs (3) dazu angepasst ist, in einem vorbestimmten Winkel in einer Herausziehrichtung (D2) des Tampon-Applikators (1) geneigt zu sein.

2. Tampon-Applikator gemäß Anspruch 1, wobei die Öffnung (10) eine Durchgangsöffnung ist.

3. Tampon-Applikator gemäß Anspruch 1 oder 2, wobei in einem Fall, in dem der Abschnitt (3B) mit großem Durchmesser gleichmäßig in drei Teile, einen vorderen Teil (A1), einen mittleren Teil (A2) und einen hinteren Teil (A3), aufgeteilt ist, die Anzahl von Öffnungen (10), die an dem hinteren Teil (A3) vorgesehen sind, größer als die Anzahl von Öffnungen (10) ist, die an dem vorderen Teil (A1) und dem mittleren Teil (A2) vorgesehen sind.

4. Tampon-Applikator gemäß Anspruch 1, 2 oder 3, wobei ein Durchmesser der Öffnung (10) 6 mm oder kleiner ist und eine Dicke des Abschnitts (3A) mit großem Durchmesser 1 mm oder weniger ist.

5. Tampon-Applikator gemäß einem der Ansprüche 1 bis 4, wobei Intervalle der Öffnungen (10) 2 mm oder mehr sind.

6. Tampon-Applikator (10), umfassend:
ein äußeres Rohr (103) zum Aufbewahren eines Tampons (102); und
ein Herausdrückelement (104), das verschiebbar in einen Öffnungsteil (106) an einem hinteren Ende des äußeren Rohrs (103) eingeführt wird und dazu fähig ist, von der Rückseite des Tampons (102) aus, der in dem äußeren Rohr (103) aufbewahrt ist, diesen zusammenzudrücken und dann den zusammengedrückten Tampon (102) aus einem Öffnungsteil (107) an einem vorderen Ende des äußeren Rohrs (103) herauszudrücken,
wobei mehrere konvexe Teile (110) an einer Außenfläche des äußeren Rohrs (103) vorgesehen sind;
**dadurch gekennzeichnet, dass**:
das äußere Rohr (103) einen Abschnitt (103A) mit großem Durchmesser zum Aufbewahren des Tampons (102) und einen Abschnitt (103B) mit kleinem Durchmesser hat, dessen Durchmesser kleiner als derjenige des Abschnitts (103A) mit großem Durchmesser ist, der mehr an einer Basisteilseite als der Abschnitt (103A) mit großem Durchmesser angeordnet ist;
die mehreren konvexen Teile (110) auf einer Außenfläche des Abschnitts (103A) mit großem Durchmesser vorgesehen sind; und
bei mindestens einem (110A, 110B) der konvexen Teile (110) dessen Höhe an einer hinteren Endseite des äußeren Rohrs (103) so ausgebildet ist, dass sie größer als dessen Höhe an einer vorderen Endseite des äußeren Rohrs (103) ist.

7. Tampon-Applikator gemäß Anspruch 6, wobei in einem Fall, in dem der Abschnitt (103A) mit großem Durchmesser gleichmäßig in drei Teile, einen vorderen Teil (A101), einen mittleren Teil (A102) und einen hinteren Teil (A103), aufgeteilt ist, die Anzahl konvexer Teile (110), die an dem hinteren Teil (A103) vorgesehen sind, größer als die Anzahl konvexer Teile (110) ist, die an dem vorderen Teil (A101) und dem mittleren Teil (A102) vorgesehen sind.

8. Tampon-Applikator gemäß Anspruch 6 oder 7, wobei mindestens einer (111) der konvexen Teile (110) in einer durchgehenden oder unterbrochenen Form ausgebildet ist und so ausgebildet ist, dass er um den Abschnitt (103A) mit großem Durchmesser herum geht.

9. Tampon-Applikator gemäß Anspruch 8, wobei mindestens einer (111) der konvexen Teile (110) in einer im Wesentlichen wellenartigen Form ausgebildet ist.

10. Tampon-Applikator gemäß einem der Ansprüche 6 bis 9, wobei eine Höhe eines konvexen Teils (112C, 113C), die an einer hintersten Endseite des äußeren Rohrs (103) vorgesehen ist, so ausgebildet ist, dass sie unter den mehreren konvexen Teilen (112A, 112B, 112C; 113A, 113B, 113C) am größten ist.

11. Tampon-Applikator gemäß einem der Ansprüche 6 bis 10, wobei die Höhe der konvexen Teile (110) 0,1 mm oder mehr und 3 mm und weniger beträgt.

12. Tampon-Applikator gemäß einem der Ansprüche 6 bis 11, wobei Intervalle zwischen den konvexen Teilen (110) 1 mm oder mehr betragen.

13. Tampon-Applikator gemäß einem der vorhergehenden Ansprüche, wobei das äußere Rohr (3; 103) aus einem Werkstoff ausgebildet ist, dessen Oberflächenenergie geringer als 37 x 10⁻³ N/m ist.

## Revendications

1. Applicateur de tampon (1) comprenant :
un tube externe (3) pour stocker un tampon (2) ; et
un élément de poussée (4) qui est inséré de manière coulissante dans une partie d'ouverture (6) au niveau d'une extrémité arrière du tube externe (3) et peut appliquer une compression sur l'arrière du tampon (2) qui est stocké dans le tube externe (3) et ensuite pousser le tampon (2) comprimé dans une partie d'ouverture (7) au niveau d'une extrémité avant du tube externe (3),
dans lequel au moins une ouverture (10) est prévue sur une face extérieure du tube externe (3) ;
**caractérisé en ce que** :
le tube externe (3) a une section de grand diamètre (3A) pour stocker le tampon (2) et une section de petit diamètre (3B) qui est inférieure du point de vue du diamètre à la section de grand diamètre (3A) qui est positionnée davantage du côté de la partie de base que la section de grand diamètre (3A) ;
la au moins une ouverture (10) est prévue sur une face extérieure de la section de grand diamètre (3A) ; et
dans la ou au moins une (10A, 10B, 10C) des ouvertures (10), sa face latérale (10A1, 10C1), au niveau d'un côté d'extrémité avant du tube externe (3), est adaptée pour s'incliner selon un angle prédéterminé dans une direction d'insertion (D1) de l'applicateur de tampon (1) et/ou sa face latérale (10B2, 10C2), au niveau d'un côté d'extrémité arrière du tube externe (3), est adaptée pour s'incliner selon un angle prédéterminé dans une direction de traction (D2) de l'applicateur de tampon (1).

2. Applicateur de tampon selon la revendication 1, dans lequel l'ouverture (10) est un trou débouchant.

3. Applicateur de tampon selon la revendication 1 ou 2, dans lequel, dans un cas dans lequel la section de grand diamètre (3B) est divisée en trois parties égales, une partie avant (A1), une partie centrale (A2) et une partie arrière (A3), le nombre d'ouvertures (10) qui sont prévues au niveau de la partie arrière (A3) est supérieur au nombre d'ouvertures (10) qui sont prévues au niveau de la partie avant (A1) et de la partie centrale (A2).

4. Applicateur de tampon selon la revendication 1, 2 ou 3, dans lequel un diamètre de l'ouverture (10) est de 6 mm ou moins et une épaisseur de la section de grand diamètre (3A) est de 1 mm ou moins.

5. Applicateur de tampon selon l'une quelconque des revendications 1 à 4, dans lequel les intervalles des ouvertures (10) sont de 2 mm ou plus.

6. Applicateur de tampon (10) comprenant :
un tube externe (103) pour stocker un tampon (102) ; et
un élément de poussée (104) qui est inséré de manière coulissante dans une partie d'ouverture (106) au niveau d'une extrémité arrière du tube externe (103) et peut appliquer une compression sur l'arrière du tampon (102) qui est stocké dans le tube externe (103) et ensuite pousser le tampon (102) comprimé dans une partie d'ouverture (107) au niveau d'une extrémité avant du tube externe (103),
dans lequel une pluralité de parties convexes (110) sont prévues sur une face extérieure du tube externe (103) ;
**caractérisé en ce que** :
le tube externe (103) a une section de grand diamètre (103A) pour stocker le tampon (102) et une section de petit diamètre (103B), qui est inférieure du point de vue du diamètre à la section de grand diamètre (103A), qui est positionnée davantage du côté de la partie de base que la section de grand diamètre (103A) ;
la pluralité de parties convexes (110) sont prévues sur une face extérieure de la section de grand diamètre (103A) ; et
dans au moins l'une (110A, 110B) des parties convexes (110), sa hauteur au niveau du côté de l'extrémité arrière du tube externe (103) est formée pour être plus grande que sa hauteur au niveau d'un côté de l'extrémité avant du tube externe (103).

7. Applicateur de tampon selon la revendication 6, dans lequel, dans un cas dans lequel la section de grand diamètre (103A) est divisée en trois parties égales, une partie avant (A101), une partie centrale (A102) et une partie arrière (A103), le nombre de parties convexes (110) qui sont prévues au niveau de la partie arrière (A103) est supérieur au nombre de parties convexes (110) qui sont prévues au niveau de la partie avant (A101) et de la partie centrale (A102).

8. Applicateur de tampon selon la revendication 6 ou 7, dans lequel au moins l'une (111) des parties convexes (110) est formée selon une forme continue ou intermittente et est formée afin de faire le tour de la section de grand diamètre (103A).

9. Applicateur de tampon selon la revendication 8, dans lequel au moins l'une (111) des parties convexes (110) est formée selon une forme sensiblement ondulée.

10. Applicateur de tampon selon l'une quelconque des revendications 6 à 9, dans lequel une hauteur d'une partie convexe (112C, 113C) qui est prévue du côté de l'extrémité la plus en arrière du tube externe (103), est formée afin d'être la plus grande parmi la pluralité de parties convexes (112A, 112B, 112C ; 113A, 113B, 113C).

11. Applicateur de tampon selon l'une quelconque des revendications 6 à 10, dans lequel la hauteur des parties convexes (110) est de 0,1 mm ou plus et de 3 mm ou moins.

12. Applicateur de tampon selon l'une quelconque des revendications 6 à 11, dans lequel les intervalles entre les parties convexes (110) sont de 1 mm ou plus.

13. Applicateur de tampon selon l'une quelconque des revendications précédentes, dans lequel le tube externe (3; 103) est formé avec un matériau dont l'énergie de surface est inférieure à 37 x 10⁻³ N/m.
